# FASCICULE DE BREVET EUROPEEN

(11) **EP 0 869 283 B1**
(45) Date de publication et mention de la délivrance du brevet: **06.08.2003**
(21) Numéro de dépôt: 97810194.7
(22) Date de dépôt: 04.04.1997
(51) Int. Cl.: F04B 43/12, A61M 5/142

(54) **Pompe péristaltique**
Peristaltische Pumpe
Peristaltic pump

(43) Date de publication de la demande: 07.10.1998
(73) Titulaire: Péclat, Christian, 2000 Neuchâtel (CH)
(72) Inventeur: Péclat, Christian, 2000 Neuchâtel (CH)
(74) Mandataire: Ravenel, Thierry Gérard Louis

(56) Documents cités:
- EP-A- 0 026 704
- WO-A-89/07200
- US-A- 4 773 218

## Description

La présente invention concerne une pompe péristaltique, notamment pour applications médicales, comportant un corps de pompe pourvu d'une surface d'appui intérieure en arc de cercle, un rotor, des moyens d'entraînement pour faire tourner le rotor, et au moins un tuyau compressible contenant un fluide à pomper et disposé dans un plan radial le long de ladite surface d'appui, le rotor comportant un châssis rotatif portant des galets agencés pour rouler contre le tuyau en le comprimant contre la surface d'appui.

L'invention s'applique plus particulièrement, mais pas exclusivement, à de petites pompes péristaltiques portables destinées à des applications médicales, notamment des pompes portées extérieurement par un patient ou implantées dans le corps humain ou animal, pour délivrer des quantités dosées de liquide contenant des hormones, des médicaments etc. Il est connu que de telles pompes péristaltiques peuvent comporter soit un seul tuyau, soit deux tuyaux branchés en parallèle et comprimés avec un déphasage mutuel pour fournir ensemble un débit plus régulier. Des pompes ou appareils de ce genre sont décrits par exemple dans les brevets US 4 692 147 et 5 083 908.

Ces pompes connues sont entraînées généralement par un moteur électromagnétique, habituellement un moteur pas à pas ou un moteur à courant continu associé à un capteur de position ou de débit, sous le contrôle d'une unité électronique de commande qui détermine le volume de liquide à débiter en fonction du temps. Ce moteur entraîne un arbre lié au châssis du rotor. Un tel système d'entraînement comporte divers inconvénients, dont le premier est l'encombrement. Bien que toute pompe péristaltique présente par nature un certain encombrement radial, dû à la présence des galets sur la périphérie du rotor et à l'emplacement du tuyau autour du rotor, l'encombrement axial de la pompe proprement dite peut être assez faible, mais il s'y ajoute celui du moteur, qui est habituellement décalé axialement par rapport au corps de pompe. De plus, une transmission par engrenage démultiplicateur est généralement nécessaire pour permettre l'emploi d'un moteur de faible taille et produire un couple suffisant sur l'arbre du rotor. Un autre inconvénient réside dans le fait que les moteurs électromagnétiques ont un circuit magnétique et peuvent être perturbés par des champs magnétiques extérieurs, malgré un éventuel blindage. Cela empêche par exemple des examens par résonance magnétique nucléaire. Lorsque ces moteurs sont employés dans des appareils à implanter dans le corps humain ou animal, ils doivent être dépourvus de lubrifiant afin de supporter la stérilisation, sinon on doit les encapsuler sans les stériliser.

Dans la publication WO 89/07200, il est décrit une pompe péristaltique dont les moyens d'entraînement sont du type piézo-électrique. Il en résulte que ces moyens peuvent être réalisés sous une forme particulièrement légère, avec un faible encombrement axial. De plus ces moyens sont pratiquement insensibles aux influences magnétiques extérieures. Toutefois, comme les moyens d'entraînement décrits dans ce document comportent une structure rotorique interposée entre le stator vibrant et les éléments (billes ou galets) qui roulent contre le tuyau, cette construction ne permet pas d'atteindre un maximum de compacité de la pompe.

Divers types de moteurs piézo-électriques, appelés aussi moteurs ultrasoniques, sont décrits dans un article de Y. Tomikawa et al qui est mentionné ci-dessous.

La présente invention a pour objet une pompe péristaltique permettant d'éviter substantiellement les inconvénients précités. Une telle pompe est définie dans la revendication 1.

Ainsi, les moyens d'entraînement piézo-électriques de la pompe comportent avantageusement un stator vibrant disposé entre les galets et ayant une surface périphérique circulaire dont la vibration s'effectue parallèlement au plan radial selon une onde progressive, ladite vibration entraînant les galets par friction sur leur circonférence.

D'une part, il en résulte un encombrement extrêmement faible, puisque le stator occupe en quelque sorte un espace mort, à savoir la zone centrale située entre les galets, à l'intérieur de l'encombrement axial du rotor.
D'autre part, comme l'entraînement s'effectue sur la circonférence des galets et non pas sur le châssis du rotor, il n'y a pas besoin d'un arbre d'entraînement, ni d'engrenage démultiplicateur. La pompe et ses moyens d'entraînement comprennent donc peu de pièces, notamment peu de pièces mobiles et peu de paliers. L'ensemble peut fonctionner sans lubrifiant et peut donc être stérilisé sans difficulté.

Dans une forme particulièrement avantageuse de l'invention, notamment si le nombre des galets est égal ou supérieur à trois, le rotor n'a pas besoin d'être centré exactement par rapport au stator et l'on peut donc se passer de tout palier central. De préférence, le châssis du rotor est monté avec un jeu radial par rapport au stator, ce châssis étant positionné dans le plan radial et entraîné en rotation par les galets.

D'autres caractéristiques et avantages de la présente invention apparaîtront dans la description suivante de deux formes de réalisation préférées et de diverses variantes, en référence aux dessins annexés, dans lesquels :
- la figure 1 est une vue schématique en perspective d'une première forme de réalisation d'une pompe selon l'invention;
- la figure 2 est une vue agrandie en perspective de la pompe de la figure 1, dont le couvercle a été enlevé;
- la figure 3 est une vue schématique en coupe transversale de la pompe suivant la ligne III-III de la figure 2;
- la figure 4 est une vue éclatée montrant en perspective les différents composants de la pompe des figures 1 à 3;
- la figure 5 est une vue de dessous du stator vibrant de la pompe des figures 1 à 4;
- la figure 6 est une vue en coupe transversale suivant la ligne VI-VI de la figure 5;
- la figure 7 est une vue en plan d'une variante de la pompe représentée à la figure 2;
- la figure 8 est une vue partielle en plan d'une autre variante de la pompe représentée à la figure 2;
- la figure 9 est une vue schématique en coupe transversale d'une deuxième forme de réalisation d'une pompe selon l'invention;
- la figure 10 est une vue éclatée montrant en perspective les composants de la pompe de la figure 9, et
- la figure 11 est une vue en perspective du stator vibrant de la pompe de la figure 9.

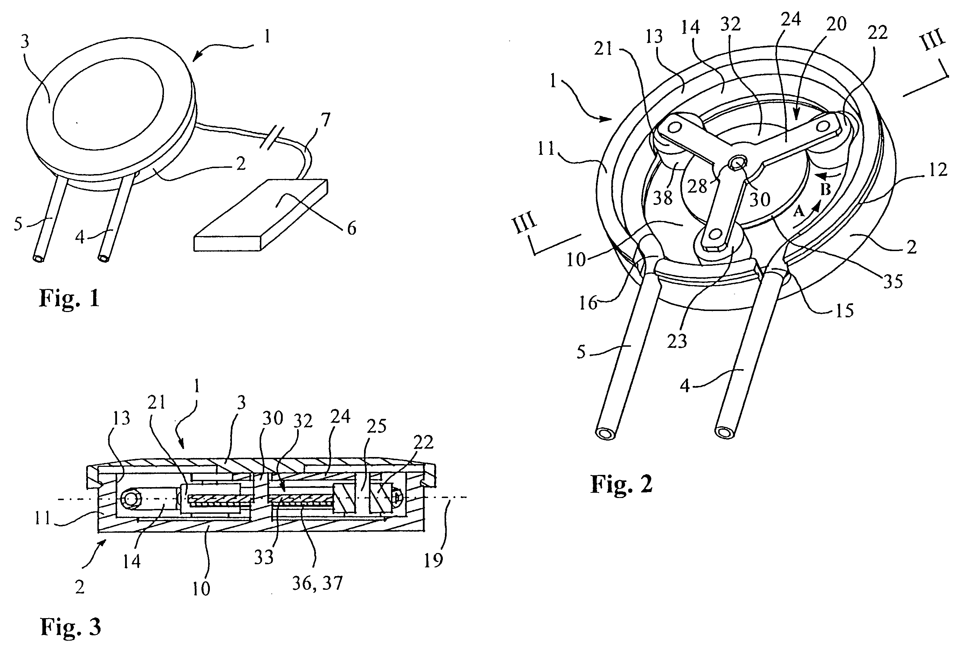

Dans la figure 1, on a représenté schématiquement une pompe péristaltique 1 selon l'invention, ayant un corps de pompe 2 dont le dessus est fermé par un couvercle 3. Le circuit du fluide pompé comporte un tube d'entrée 4 et un tube de sortie 5 qui émergent du corps 2. On a également représenté schématiquement une unité électronique 6 de commande de la pompe, sous la forme d'un élément séparé 6 relié électriquement à la pompe par un câble de transmission 7, mais il est aussi envisageable de loger l'unité 6 à l'intérieur du corps 2. Cette unité 6 comprend un dispositif électronique de commande, pour gérer le fonctionnement de la pompe 1 en fonction du temps, et une source d'énergie électrique, par exemple une pile, pour alimenter ce dispositif et les moyens d'entraînement de la pompe. Comme on l'a dit plus haut, l'ensemble représenté à la figure 1 est de préférence destiné à être porté par un patient ou implanté dans le corps d'un être humain ou animal. Dans une telle application, la pompe 1 peut avoir un diamètre de quelques centimètres, par exemple environ 4 cm, et une hauteur de l'ordre de 1 à 2 cm.

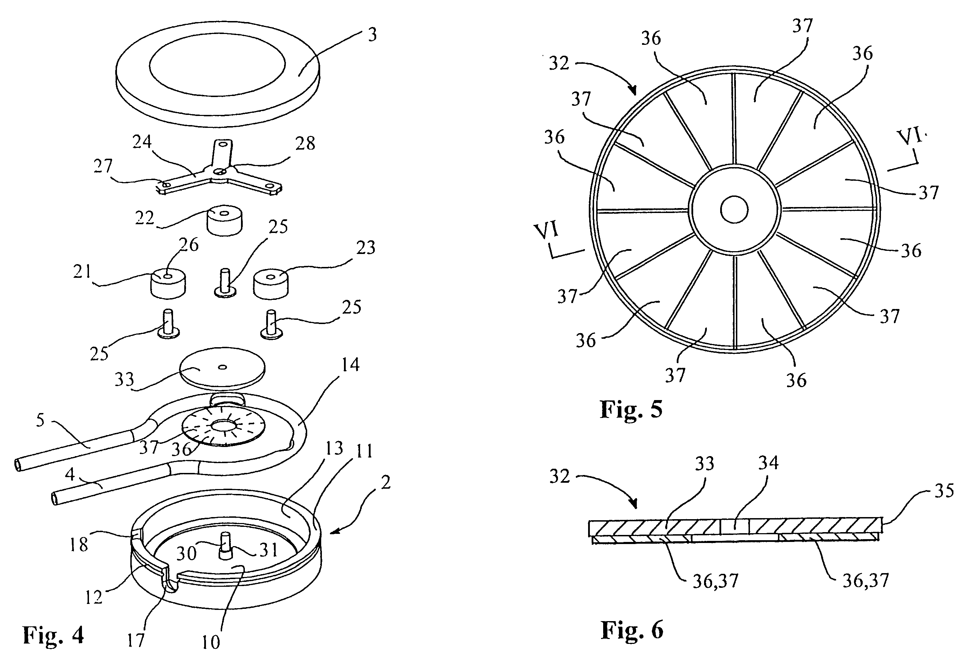

Les figures 2 à 6 représentent plus en détails une première forme de réalisation de la pompe 1. Le corps 2 a la forme générale d'une cuvette cylindrique de faible hauteur, comprenant un fond 10 et une paroi périphérique cylindrique 11. Sur la surface extérieure de la paroi 11 est ménagée une rainure 12 pour l'accrochage du couvercle 3. A l'intérieur, la paroi 11 présente une surface d'appui cylindrique 13 le long de laquelle est placé le tuyau compressible classique 14 de la pompe péristaltique. Le tuyau 14 a deux parties coudées 15 et 16 raccordées respectivement aux tubes d'entrée 4 et de sortie 5 et fixées dans des ouvertures 17 et 18 (figure 4) de la paroi 11 du corps 2. De préférence, les parties 4, 5, 14, 15 et 16 sont faites d'une seule pièce. Le tuyau 14 est appuyé librement contre la surface 13 et s'étend sur plus de la moitié de la circonférence intérieure du corps 2, de préférence sur environ 270°.

De manière connue, le tuyau 14 est comprimé contre la surface d'appui 13 au moyen de trois galets rotatifs 21, 22 et 23 qui se déplacent comme l'indique la flèche A, suivant une trajectoire circulaire autour du centre de la pompe, et roulent sur le tuyau 14 en tournant sur eux-mêmes comme l'indique la flèche B. Le déplacement suivant A produit un pompage volumétrique par déplacement du fluide contenu dans le tuyau 14 entre deux galets successifs. Le débit de sortie de la pompe est proportionnel au chemin parcouru par les galets suivant la flèche A.

Les galets 21 à 23 font partie d'un rotor 20 de la pompe, comportant un châssis rotatif plat 24 ayant, dans cet exemple, la forme d'une étoile à trois bras, mais pouvant avoir toute autre forme appropriée, telle que celle d'un disque. Comme le montre la figure 4, le galet 21 est monté de manière rotative sur l'extrémité d'un des bras du châssis 24 au moyen d'une cheville 25 passant librement à travers un trou central 26 du galet 21, cette cheville étant fixée dans un trou correspondant 27 du châssis 24. Les deux autres galets 22 et 23 sont montés de la même façon sur les autres bras du châssis 24. Celui-ci comporte un trou central 28.

Le corps 2 présente en outre sur son fond 10 un tenon central 30 ayant deux diamètres successifs pour former un épaulement 31. La hauteur du tenon 30 est approximativement égale à celle de la paroi 11 du corps, de sorte que le tenon passe à travers le trou 28 et que le couvercle 3 peut s'appuyer sur le sommet du tenon. Ce tenon sert à supporter un stator vibrant 32 qui est raccordé électriquement à l'unité de commande 6 pour constituer des moyens d'entraînement piézo-électriques de la pompe 1.

Comme on le voit plus particulièrement dans les figures 3 à 6, le stator vibrant 32 comporte d'une part un résonateur 33 en forme de disque, ayant un trou central 34 et une surface périphérique circulaire 35, et d'autre part une série d'éléments piézo-électriques 36, 37 en forme de secteurs qui ont tous la même taille et sont collés contre la face inférieure du résonateur 33, les éléments 37 étant disposés en alternance avec les éléments 36. Le résonateur 33 est fixé au corps 2 par chassage du tenon 30 dans le trou 34 jusqu'à ce que le résonateur bute contre l'épaulement 31. Il s'étend alors dans un plan radial 19 situé de préférence au niveau du tuyau 14.

De préférence, chaque élément piézo-électrique 36, 37 est en réalité une zone active en forme de secteur d'une plaque commune annulaire de céramique piézo-électrique, par exemple du type PZT. L'étendue de chacune de ces zones est définie par une paire d'électrodes raccordées à l'unité de commande 6 et disposées de part et d'autre de la plaque de façon qu'une tension électrique appliquée aux électrodes provoque une déformation de la plaque de céramique dans son plan, c'est-à-dire une déformation par extension et raccourcissement à la fréquence de la tension électrique alternative. Ces déformations sont transmises au résonateur 33. Bien entendu, la fréquence de la tension électrique est choisie de manière à coïncider avec une fréquence propre d'un mode de résonance par extension du résonateur 33. La fréquence de résonance dépend de la forme, des dimensions et du matériau du résonateur, ainsi que du choix du mode propre de résonance. En pratique, cette fréquence est choisie dans une gamme allant d'environ 20 kHz (seuil d'audition) à environ 300 kHz. En fait, c'est seulement sur la face inférieure de la plaque de céramique que les électrodes sont en forme de secteurs. Sur l'autre face, du côté du résonateur 33, il y a une seule électrode de masse continue en forme de disque. Le résonateur lui-même peut être relié électriquement à l'électrode de masse ou constituer cette électrode.

Tous les éléments 36 sont connectés en parallèle pour recevoir une première tension d'excitation alternative, tandis que tous les éléments 37 sont également connectés en parallèle pour recevoir une seconde tension d'excitation alternative ayant la même grandeur et la même fréquence que la première, mais déphasée d'un quart de période. Il en résulte que le résonateur 33 vibre par extension dans son plan sous la forme d'une onde progressive qui chemine autour du stator 32 dans le sens opposé à la flèche A de la figure 2. Pendant cette vibration, chaque point de la surface périphérique 35 du résonateur décrit une trajectoire elliptique dans le plan du résonateur, ce qui entraîne par friction une rotation de chaque galet 21, 22, 23 dans le sens de la flèche B. Les électrodes et leurs liaisons électriques avec l'unité de commande 6 ne sont pas représentées, afin de clarifier les dessins.

Des éléments vibrants de ce genre et leurs applications à des moteurs piézo-électriques, appelés aussi moteurs ultrasoniques, sont décrits notamment dans un article de Y. Tomikawa, T. Ogasawara et T. Takano, intitulé "Ultrasonic Motors - Constructions / characteristics / applications", publié dans Ferroelectrics, 1989, Vol. 91, pp. 163-178 (Gordon and Breach Science Publishers), qui est incorporé ici par référence.

Il faut noter que le stator vibrant 32 pourrait être constitué simplement d'un disque circulaire ou annulaire de céramique piézo-électrique, équipé d'électrodes appropriées sur ses deux faces, sans le résonateur en aluminium 33.

D'une manière générale, l'onde progressive présente un ventre et une paire de noeuds pour chaque paire d'éléments 36 (ou 37), c'est-à-dire que le stator 32 doit comporter au moins quatre éléments piézo-électriques. Pour entraîner simultanément les galets, il est préférable que le nombre de ventres et de paires de noeuds soit égal à celui des galets. Dans l'exemple préféré représenté aux figures 5 et 6, il est prévu trois paires d'éléments 36 et trois paires d'éléments 37 afin de produire une onde ayant trois ventres décalés de 120° pour entraîner simultanément les trois galets 21, 22 et 23, ce qui réduit les vibrations et les efforts dans le rotor.

On notera que dans la position représentée à la figure 2, les galets 21 et 22 sont pressés contre le tuyau 14 par appui contre le stator vibrant 32, la circonférence 38 de chaque galet étant appuyée directement contre la surface périphérique 35 du stator par l'élasticité de la matière du tuyau. Ainsi, ces deux galets sont guidés positivement par le stator le long d'une trajectoire en arc de cercle. Par contre, le troisième galet 23 ne roule pas contre le tuyau 14 à ce moment-là, de sorte qu'il n'a pas tendance à avancer, même s'il est entraîné en rotation par le stator. Il est alors entraîné dans le sens de la flèche A par la rotation du châssis 24, dont la position dans son propre plan est déterminée à chaque instant par les deux galets 21 et 22 roulant contre le tuyau. La fonction essentielle du châssis 24 est donc uniquement de maintenir la distance angulaire entre les galets, tandis que leur trajectoire est définie par d'autres moyens. Par conséquent, le rotor 20 n'a pas besoin d'être parfaitement centré par rapport au stator et c'est pourquoi le trou central 28 du châssis 24 a un diamètre un peu plus grand que celui du tenon 30, afin de ménager un jeu radial entre ces deux pièces. Le rotor 20 n'a donc pas d'arbre ni de palier central, les seuls paliers étant formés par les chevilles 25 qui portent les galets. Contrairement aux pompes péristaltiques classiques, le châssis 24 et les chevilles 25 n'ont pas à transmettre de couple moteur des moyens d'entraînement aux galets.

Le résonateur 33 est de préférence en aluminium, mais il peut être fait de toute autre matière ayant des caractéristiques mécaniques appropriées, notamment quant à son module d'élasticité. Le tuyau 14 est de préférence en élastomère. Les autres pièces de la pompe peuvent être faites de toute matière appropriée à l'utilisation prévue, notamment en matière synthétique pour réduire le poids de la pompe et son coût de fabrication. La pompe peut ainsi être entièrement amagnétique.

Dans la figure 3, on remarque que la pompe 1 est extrêmement compacte, grâce à la simplicité de son rotor et grâce à la position avantageuse du stator 32 dans l'espace habituellement inutilisé à l'intérieur de la trajectoire des galets, si bien que l'encombrement de la pompe est déterminé essentiellement par les dimensions du rotor.

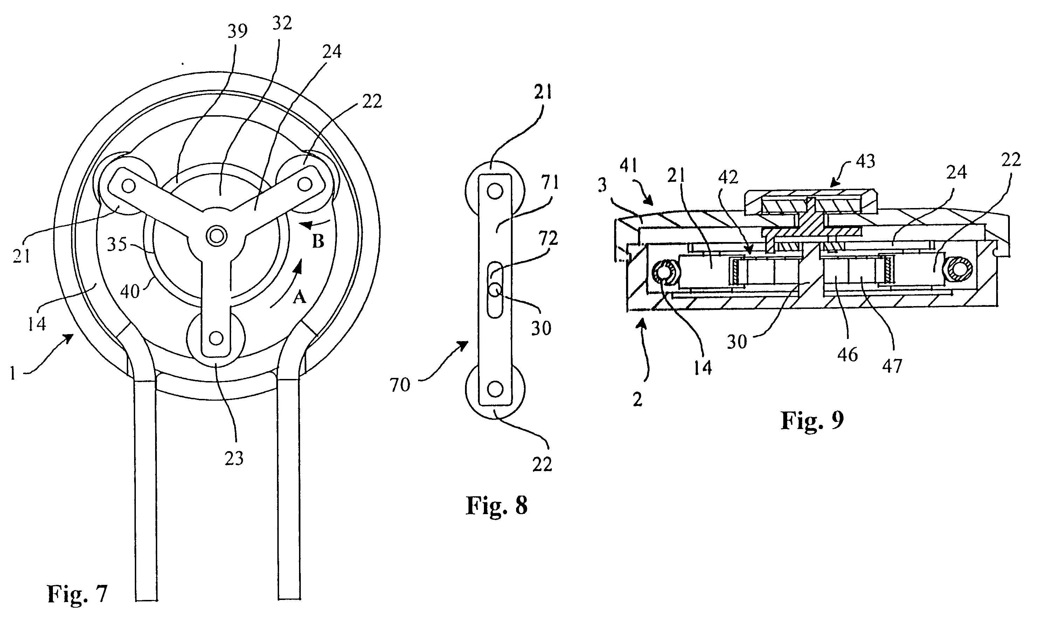

La figure 7 représente une variante de la pompe 1 décrite ci-dessus, où la différence est que les galets 21, 22 et 23 ne s'appuient pas directement contre la surface périphérique 35 du stator 32, bien qu'ils soient aussi entraînés au moyen de l'onde progressive faisant vibrer cette surface. Un élément annulaire rotatif 39 entoure le stator 32 en étant serré contre la surface périphérique 35 de celui-ci par des moyens élastiques (non représentés). Par exemple, l'élément 39 peut être divisé en deux ou plusieurs segments séparés par de petits intervalles et tirés les uns contre les autres par des ressorts ou par une bande élastique entourant l'élément 39. Un moteur piézo-électrique de ce genre est également décrit dans la publication de Tomikawa et al. citée plus haut.

L'élément annulaire 39 est aussi pressé contre le stator vibrant 32 par les galets 21 et 22 poussés par l'élasticité du tuyau 14. L'onde progressive l'entraîne dans le sens de la flèche A, si bien que sa surface périphérique 40 fait tourner les galets par friction sur leur circonférence. Par rapport à l'exemple décrit plus haut, cette construction permet de choisir plus librement les matériaux, notamment celui des galets puisque ceux-ci ne coopèrent pas directement avec la surface vibrante. Par exemple, l'élément 39 peut comporter un premier matériau relativement dur sur sa surface intérieure s'appuyant contre le stator 32, et un autre matériau moins dur et à plus grand coefficient de friction sur sa surface extérieure coopérant avec les galets 21 à 23. Pour le reste, la construction et le fonctionnement sont les mêmes que dans l'exemple précédent.

Dans une variante, on pourrait aussi envisager que l'élément annulaire 39 entraîne chaque galet par une transmission à engrenage, ce qui éviterait tout risque de glissement entre eux.

La figure 8 représente une autre variante de la réalisation selon les figures 1 à 6, où le rotor 20 est remplacé par un rotor 70 ne comportant que deux galets 21 et 22 diamétralement opposés. Le châssis 71 du rotor n'a alors que deux bras diamétralement opposés et il est guidé latéralement pour entraîner le galet ne touchant pas le tuyau. Pour cela, il présente un trou central 72 en forme de fente diamétrale de largeur égale au diamètre supérieur du tenon 30 engagé dans ce trou. Il en résulte un jeu radial du rotor par rapport au stator dans la direction du diamètre passant par les axes des deux galets 21 et 22, ce qui permet à chaque galet de suivre la trajectoire imposée par la périphérie du stator vibrant sans créer d'effort substantiel dans le châssis 71. Une telle variante à deux galets a l'avantage de permettre l'utilisation d'un mode de résonance d'ordre moins élevé, donc avec de plus grandes déformations du stator et une réduction de la fréquence d'excitation et du nombre d'électrodes. Elle est aussi applicable aux autres réalisations décrites ici.

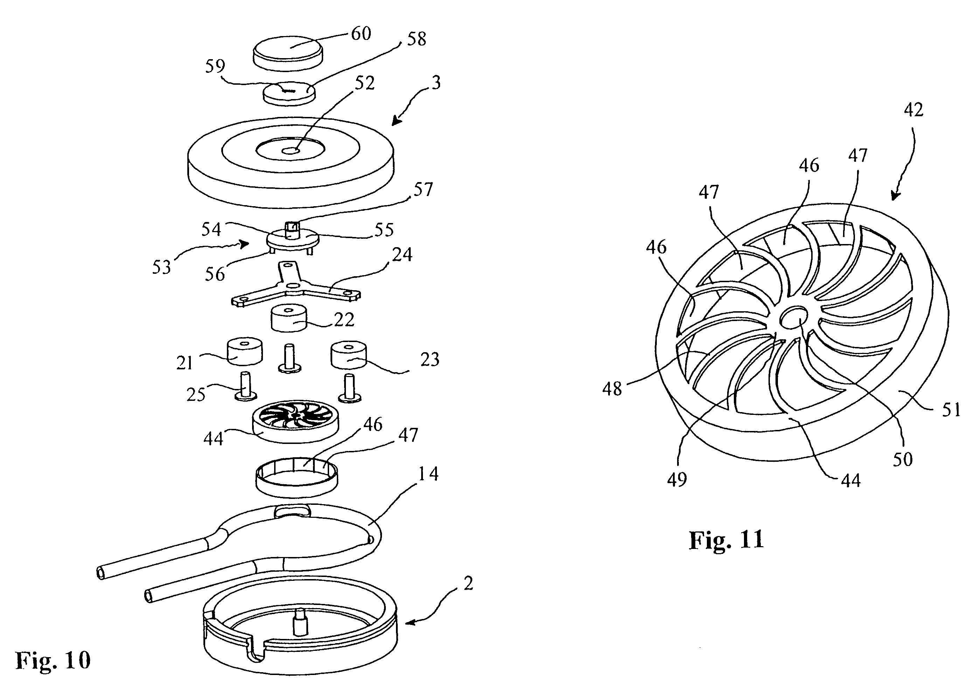

Les figures 9 à 11 représentent une deuxième forme de réalisation d'une pompe péristaltique 41 selon l'invention, qui se différencie de la pompe 1 décrite plus haut essentiellement par un stator vibrant 42 de forme différente. La pompe 41 comporte en outre un capteur de position angulaire 43 qui peut aussi être incorporé à la pompe 1 et à n'importe quel mode de réalisation de l'invention. Les autres éléments de la pompe 41 peuvent être semblables à ceux de la pompe 1, portent les mêmes numéros de référence et ne seront pas décrits en détail ci-dessous.

Le stator vibrant 42 comprend un résonateur formé essentiellement d'une bague vibrante cylindrique 44, et une rangée circulaire d'éléments piézo-électriques 46 et 47 disposés en alternance sur la face intérieure de la bague 44 et fixés à celle-ci pour la mettre en vibration par extension dans un plan radial selon une onde progressive, d'après le même principe décrit en référence au stator vibrant 32 de la pompe 1. Le nombre des éléments 46 et 47 peut être le même que celui des éléments 36 et 37, et ces éléments peuvent aussi être constitués chacun par une zone active délimitée par les électrodes sur une unique pièce de céramique piézo-électrique ayant ici une forme cylindrique. Pour supporter la bague vibrante 44, le résonateur comporte une série de bras flexibles 48 sensiblement radiaux, reliant cette bague à un moyeu 49 pourvu d'un trou central 50 pour être chassé sur le tenon central 30 du corps 2. La bague 44 présente une surface périphérique cylindrique 51 contre laquelle les galets 21 à 23 sont pressés par l'élasticité de la matière du tuyau compressible 14. Cette surface 51 joue le même rôle que la surface périphérique 35 de la pompe 1, mais elle offre une plus grande surface de contact avec les galets.

D'autre part, un homme du métier comprendra qu'en comparaison avec le résonateur 33 en forme de plaque, le résonateur 44 en forme de bague offre une plus grande liberté de choix dans les dimensions, donc aussi dans les fréquences propres de vibration du résonateur. Cela peut être utile notamment pour éviter l'emploi de fréquences d'excitation trop élevées quand le stator a un petit diamètre. En outre, il reste de la place dans la région centrale pour y loger l'unité de commande 6 s'il le faut.

Par ailleurs on notera que la variante de la première forme de réalisation, comportant un élément annulaire rotatif 39 selon la figure 7, est aussi applicable à la forme de réalisation illustrée par les figures 9 à 11.

Le capteur de position angulaire 43 est monté sur le couvercle 3 du corps de pompe, au-dessus d'un alésage central 52 du couvercle. Il est lié en rotation au châssis 24 du rotor au moyen d'un élément de transmission 53 ayant un moyeu central 54 monté à rotation dans l'alésage 52. L'élément 53 comporte un disque 55 logé entre le couvercle 3 et le châssis 24 et pourvu de griffes 56 qui embrassent les bras du châssis 24 pour que l'élément 53 tourne conjointement avec le rotor. Le moyeu 54 a une tête rectangulaire 57 destinée à entraîner le capteur de rotation.

Le capteur 43 peut être de n'importe quel type connu, analogique ou numérique, capable de délivrer un signal électrique représentatif de la position angulaire du rotor par rapport au couvercle 3. Dans cet exemple, il s'agit d'un capteur analogique de type résistif, comportant un codeur angulaire résistif en forme de disque 58 coopérant avec trois bornes solidaires du couvercle. La tête rectangulaire 57 de l'élément 53 est engagée dans une fente correspondante 59 au centre du disque 58 pour faire tourner celui-ci avec le rotor. Le disque 58 est retenu et guidé par un capuchon circulaire 60 scellé au couvercle 3. Le capteur 43 délivre son signal de sortie à l'unité de commande 6 par une liaison électrique qui n'est pas représentée. L'unité de commande peut ainsi mesurer en temps réel le volume de liquide débité par la pompe péristaltique.

Un homme du métier pourra envisager de multiples modifications et variantes dans les exemples décrits ci-dessus, sans sortir du cadre de la présente invention. En particulier on notera que les mêmes principes de construction et d'entraînement sont applicables à une pompe péristaltique comportant deux tuyaux en parallèle. Dans un tel cas, les deux tuyaux peuvent être superposés pour être comprimés par les mêmes galets, par exemple si la surface d'appui 13 de l'un des tuyaux est décalée angulairement par rapport à la surface d'appui de l'autre tuyau, selon le principe décrit dans le brevet US 5 083 908 cité plus haut. Ces galets peuvent être entraînés par un unique stator vibrant.

## Revendications

1. Pompe péristaltique, notamment pour applications médicales, comportant un corps de pompe (2) pourvu d'une surface d'appui intérieure (13) en arc de cercle, un rotor (20, 70), des moyens d'entraînement pour faire tourner le rotor, et au moins un tuyau compressible (14) contenant un fluide à pomper et disposé dans un plan radial (19) le long de ladite surface d'appui, le rotor comportant un châssis rotatif (24, 71) portant des galets (21-23) agencés pour rouler contre le tuyau (14) suivant une trajectoire circulaire en comprimant le tuyau contre la surface d'appui, les moyens d'entraînement étant du type piézo-électrique et comportant un stator vibrant (32, 42) disposé à l'intérieur de la trajectoire des galets (21-23) et ayant une surface périphérique circulaire (35, 51) dont la vibration s'effectue parallèlement au plan radial (19) selon une onde progressive, la pompe étant **caractérisée en ce que** ladite vibration entraîne les galets (21-23) par friction sur leur circonférence (38), le châssis rotatif (24, 71) du rotor étant entraîné par le déplacement des galets le long de leur trajectoire.

2. Pompe selon la revendication 1, **caractérisée en ce que** les galets sont pressés contre le tuyau (14) par appui contre le stator vibrant (32, 42).

3. Pompe selon la revendication 2, **caractérisée en ce que** les galets sont directement appuyés contre la surface périphérique (35, 51) du stator vibrant.

4. Pompe selon la revendication 2, **caractérisée en ce qu'**un élément annulaire rotatif (39) est intercalé entre la surface périphérique du stator vibrant et la circonférence de chaque galet, de sorte que ledit élément annulaire est entraîné par le stator vibrant et entraîne les galets sur leur circonférence.

5. Pompe selon la revendication 3 ou 4, **caractérisée en ce que** le stator vibrant (32) comporte un résonateur (33) formé d'une plaque vibrante s'étendant suivant ledit plan radial et équipée d'éléments piézo-électriques (36, 37) sur au moins une de ses faces.

6. Pompe selon la revendication 3 ou 4, **caractérisée en ce que** le stator vibrant (42) comporte un résonateur formé d'une bague vibrante cylindrique (44) équipée d'éléments piézo-électriques (46, 47) sur sa face intérieure.

7. Pompe selon la revendication 1, **caractérisée en ce que** l'onde progressive présente un nombre de noeuds qui est le double du nombre de galets.

8. Pompe selon l'une des revendications 1 à 7, **caractérisée en ce que** le nombre de galets (21-23) est égal ou supérieur à trois et **en ce que** le châssis (24) du rotor est monté avec un jeu radial par rapport au stator, ledit châssis étant positionné dans le plan radial et entraîné en rotation par les galets.

9. Pompe selon l'une des revendications 1 à 7, **caractérisée en ce que** le nombre de galets (21-22) est égal à deux, les galets étant diamétralement opposés, et **en ce que** le châssis (71) du rotor est monté avec un jeu radial par rapport au stator dans la direction du diamètre passant par les galets, mais est guidé perpendiculairement à ladite direction.

10. Pompe selon la revendication 1, **caractérisée en ce qu'**elle comporte un capteur de position (43) agencé pour détecter la position angulaire du châssis rotatif (24, 71) et délivrer un signal correspondant à des moyens de commande (6) de la pompe.

## Patentansprüche

1. Peristaltische Pumpe, insbesondere für medizinische Anwendungen, die einen Pumpenkörper (2), der mit einer kreisbogenförmigen inneren Abstützoberfläche (13) versehen ist, einen Rotor (20, 70), Antriebsmittel zum Drehen des Rotors und wenigstens einen komprimierbaren Schlauch (14), der ein zu förderndes Fluid enthält und in einer radialen Ebene (19) längs der Abstützoberfläche angeordnet ist, umfaßt, wobei der Rotor einen drehbaren Rahmen (24, 71) umfaßt, der Rollen (21-23) trägt, die so angeordnet sind, daß sie längs einer kreisförmigen Bahn an dem Schlauch (14) entlang rollen, wobei sie den Schlauch gegen die Abstützoberfläche zusammendrücken, wobei die Antriebsmittel vom piezoelektrischen Typ sind und einen schwingenden Stator (32, 42) umfassen, der innerhalb der Bahn der Rollen (21-23) angeordnet ist und eine kreisförmige Umfangsoberfläche (35, 51) besitzt und dessen Schwingungen parallel zur radialen Ebene (19) gemäß einer Wanderwelle erfolgen, wobei die Pumpe **dadurch gekennzeichnet ist, daß** die Schwingungen die Rollen (21-23) an ihrer Abrollfläche (38) reibschlüssig antreiben, wobei der drehbare Rahmen (24, 71) des Rotors durch die Verlagerung der Rollen längs ihrer Bahn angetrieben wird.

2. Pumpe nach Anspruch 1, **dadurch gekennzeichnet, daß** die Rollen durch Abstützen an dem schwingenden Stator (32, 42) gegen den Schlauch (14) gepreßt werden.

3. Pumpe nach Anspruch 2, **dadurch gekennzeichnet, daß** sich die Rollen direkt an der Umfangsoberfläche (35, 51) des schwingenden Stators abstützen.

4. Pumpe nach Anspruch 2, **dadurch gekennzeichnet, daß** zwischen die Umfangsoberfläche des schwingenden Stators und die Abrollfläche jeder Rolle ein drehbares ringförmiges Element (39) eingefügt ist, derart, daß das ringförmige Element durch den schwingenden Stator angetrieben wird und die Rollen an ihrer Abrollfläche antreibt.

5. Pumpe nach Anspruch 3 oder 4, **dadurch gekennzeichnet, daß** der schwingende Stator (32) einen Resonator (33) umfaßt, der aus einer schwingenden Platte gebildet ist, die sich längs der radialen Ebene erstreckt und wenigstens auf einer ihrer Flächen mit piezoelektrischen Elementen (36, 37) ausgerüstet ist.

6. Pumpe nach Anspruch 3 oder 4, **dadurch gekennzeichnet, daß** der schwingende Stator (42) einen Resonator umfaßt, der aus einem zylindrischen schwingenden Ring (44) gebiidet ist, der an seiner inneren Fläche mit piezoelektrischen Elementen (46, 47) ausgerüstet ist.

7. Pumpe nach Anspruch 1, **dadurch gekennzeichnet, daß** die Wanderwelle eine Anzahl von Knoten aufweist, die gleich der doppelten Anzahl der Rollen ist.

8. Pumpe nach einem der Ansprüche 1 bis 7, **dadurch gekennzeichnet, daß** die Anzahl der Rollen (21-23) gleich oder größer als drei ist und daß der Rahmen (24) des Rotors in bezug auf den Stator mit radialem Spiel angebracht ist, wobei der Rahmen in der radialen Ebene positioniert ist und durch die Rollen rotatorisch angetrieben wird.

9. Pumpe nach einem der Ansprüche 1 bis 7, **dadurch gekennzeichnet, daß** die Anzahl der Rollen (21-22) gleich zwei ist, wobei sich die Rollen einander diametral gegenüber befinden, und daß der Rahmen (71) des Rotors in bezug auf den Stator in der Richtung des durch die Rollen verlaufenden Durchmessers mit einem radialen Spiel versehen ist, jedoch senkrecht zu dieser Richtung geführt wird.

10. Pumpe nach Anspruch 1, **dadurch gekennzeichnet, daß** sie einen Positionssensor (43) umfaßt, der so beschaffen ist, daß er die Winkelposition des drehbaren Rahmens (24, 71) erfaßt und ein entsprechendes Signal an Steuermittel (6) der Pumpe liefert.

## Claims

1. Peristaltic pump, in particular for medical applications, including a pump body (2) having an interior bearing surface (13) in the form of a circular arc, a rotor (20, 70), drive means to turn the rotor, and at least one compressible tube (14) containing a fluid to be pumped and arranged in a radial plane (19) along the length of the bearing surface, the rotor including a rotary chassis (24, 71) carrying rollers (21-23) adapted to roll against the tube (14) following a circular trajectory while compressing the tube against the bearing surface, the drive means being of the piezoelectric type and including a vibrating stator (32, 42) disposed inside the trajectory of the rollers (21-23) and having a circular peripheral surface (35, 51), the vibration of which is achieved parallel to the radial plane (19) in accordance with a progressive wave, the pump being **characterized in that** said vibration drives the rollers (21-23) by friction on their circumference (38), the rotary chassis (24, 71) of the rotor being driven by the movement of the rollers along their trajectory.

2. Pump according to claim 1, **characterized in that** the rollers are pressed against the tube (14) by pressure against the vibrating stator (32, 42).

3. Pump according to claim 2, **characterized in that** the rollers are directly pressed against the peripheral surface (35, 51) of the vibrating stator.

4. Pump according to claim 2, **characterized in that** a rotating ring-shaped element (39) is inserted between the peripheral surface of the vibrating stator and the circumference of each roller, whereby the ring-shaped element is driven by the vibrating stator and drives the rollers on their circumference.

5. Pump according to claim 3 or 4, **characterized in that** the vibrating stator (32) includes a resonator (33) formed of a vibrating plate extending along said radial plane and equipped with piezoelectric elements (36, 37) on at least one of its faces.

6. Pump according to claim 3 or 4, **characterized in that** the vibrating stator (42) includes a resonator formed by a cylindrical vibrating ring (44) equipped with piezoelectric elements (46, 47) on its inner face.

7. Pump according to claim 1, **characterized in that** the number of nodes of the progressive wave is twice the number of rollers.

8. Pump according to any of claims 1 to 7, **characterized in that** the number of rollers (21-23) is equal or greater than three and **in that** the chassis (24) of the rotor is mounted with a radial play with respect to the stator, said chassis being positioned in the radial plane and driven in rotation by the rollers.

9. Pump according to any of claims 1 to 7, **characterized in that** the numbers of rollers (21-22) is two, the rollers being diametrically opposed to each other, and **in that** the chassis (71) of the rotor is mounted with a radial play with respect to the stator in the direction of the diameter passing through the rollers, but is guided perpendicularly to said direction.

10. Pump according to claim 1, **characterized in that** it includes a position sensor (43) adapted to detect the angular position of the rotary chassis (24, 71) and deliver a corresponding signal to means (6) for controlling the pump.
